# EUROPEAN PATENT SPECIFICATION

(11) **EP 3 391 873 B1**
(45) Date of publication and mention of the grant of the patent: **10.06.2026**
(21) Application number: 16852958.4
(22) Date of filing: 18.01.2016
(51) Int. Cl.: A61K 8/41, A61K 8/362, A61Q 5/00

(54) **METHOD AND KIT FOR TREATING HAIR**
VERFAHREN UND KIT ZUR HAARBEHANDLUNG
PROCÉDÉ ET KIT DE TRAITEMENT CAPILLAIRE

(30) Priority: 08.10.2015 CN 201510645964
(43) Date of publication of application: 24.10.2018
(73) Proprietor: LIW Patent Company limited by guarantee, Dublin 3 (IE)
(72) Inventor: WAGNER, Sabine, Werrington County, New South Wales 2747 (AU)
(74) Representative: Meyer-Dulheuer MD Legal Patentanwälte PartG mbB
(86) International application number: PCT/CN2016/000029
(87) International publication number: WO 2017/059646

(56) References cited:
- CN-A- 102 100 643
- DE-A1- 10 051 774
- DE-B- 1 220 969
- GB-A- 1 186 101
- US-A- 5 628 991
- US-A1- 2015 034 117
- DATABASE GNPD [online] MINTEL; 2 June 2008 (2008-06-02), ANONYMOUS: "Relaxer System", XP055950006, retrieved from https://www.gnpd.com/sinatra/recordpage/922567/ Database accession no. 922567
- T ITOU ET AL: "Study of the interaction between hair protein and organic acid that improves hair-set durability by near-infrared spectroscopy", JOURNAL OF COSMETIC SCIENCE 57(2), 1 January 2006 (2006-01-01), pages 139 - 151, XP055619711, Retrieved from the Internet <URL:http://www.ncbi.nlm.nih.gov/pubmed/16688377> [retrieved on 20190909]

## Description

### FIELD OF THE INVENTION

The present invention relates to a method and kit for strengthening hair, comprising providing a component capable of crosslinking an amino acid in a keratin fiber and mixing the ingredient with a commercially available hair dyeing or bleaching agent.

### BACKGROUND OF THE INVENTION

Frequent bleaching of hair, hair dyeing, perming, and frequent use of surfactants or other active ingredients to wash the hair can cause damage to the hair structure. The hair will become brittle and lose its luster. When combing the hair, the hair is knotted due to static electricity, making it difficult to comb the hair.

Hair care ingredients that make carding easy are very important. Such care ingredients are typically applied to the still moist hair in the form of a clear hair care cleanser or in the form of an emulsion, which is applied for a few minutes to an hour and then washed off with water.

The hair care method described above is only effective for dry hair and porous hair. For hair that is quickly oiled, its effect is not significant, because the hair is more oily after use, resulting in a hair style that does not last long. The long-term hair style depends on the disulfide bridge, the disulfide bond is unstable, and is reduced to a sulfhydryl group under reducing conditions. There have been many attempts to oxidize a sulfhydryl group to a disulfide bridge by adding an oxidizing agent. These methods are used to improve the stability of perm. Many patents and patent applications describe how to re-establish disulfide bonds, such as US 9,095,518, US 2015034119A1, US 201537270A1, US 201537271A1 and WO 2015017768. However, the techniques described in these documents still have a lot of room for improvement.

There have also been many attempts in the prior art to overcome the drawbacks of known hair care techniques by the addition of amino acids, such as a mixture of various amino acids and vitamins, as a conditioner (US-PS 4201235). However, preparing such a multivitamin and amino acid mixture solution is cumbersome and expensive.

The use of keratin hydrolysate and citric acid in a neutralizing shampoo is described in "Cosmetic and Cosmetics, Vol 98 (1983), S. 59-68". However, this shampoo has only a very low hair care function and can cause the hair to be extremely dry. Therefore, it takes one or more uses of the conditioner after shampooing.

The document "W. Fassbender, Perfume & Cosmetics, 39(1), S. 11-16 (1958)" discloses that a mixture containing 18 to 22 amino acids can be used in skin care and hair care products. This mixture can be obtained by isolating the hydrolysate of the native protein and subsequent washing. Therefore, it is difficult to achieve the proportion of ingredients required for cosmetics.

### Summary of the invention

The present invention relates to a method according to appended claims 1 to 13 for strengthening hair and/or repairing hair comprising the steps of: (a) applying a formulation (a) to the hair having at least a bifunctional Bronsted base of the formula XRY, wherein X and Y are proton acceptors, R is an organic spacer having 1 to 20 carbon atoms, 0 to 5 oxygen atoms, and 0 to 5 nitrogen atoms, and the molecular weight of XRY is less than 500 g/mol, as defined in appended claim 1, and it is in the hair for 1 to 45 minutes, (b) optionally cleaning, and/or drying the hair, (c) applying an acidic formulation (b) to the hair, the formulation having an at least bifunctional organic acid as defined in claim 1 with which the amino group in the hair reacts, and leaving it on the hair for 1 to 45 minutes, (d) optionally washes, and/or dries the hair, characterized in that the pH of the formula (a) is 7 to 12 and is alkaline and has a pH greater than 7, and in that the pH of formula (b) is from 1.5 to 7 and is acidic and has a pH of less than 7. Optionally, the strong hair formula in step (a) is mixed with a commercially available dye or bleach prior to use to the hair. If the strong hair formula in step (a) is not mixed with a commercially available dye or bleach prior to use to the hair, the above method may be modified to advance step (c) to before step (a).

### Detailed description of the invention

In the present document, all the examples are included, which are mainly defined below unless otherwise stated. All percentages are by weight of the total ingredients. All ratios are by weight. "Parts", for example, 1 part X, 3 parts Y, is a weight ratio. "QS" or "QSP" means the quantity is 100% or 100g. +/- is the standard deviation. All ranges are inclusive and combinable. The number of valid digits does not have a limiting effect on the number indicated, nor does it represent the accuracy of the measurement. All numbers are described by "about". All measurements were made at 25 ° C and ambient conditions. "Environmental conditions" refers to 1 atmosphere, 50% relative humidity. "Relative humidity" refers to the percentage of moisture content in the air relative to the same temperature and saturated humidity levels at atmospheric pressure. The relative humidity can be measured with a hygrometer. "min" means minutes, "mol" means moles, and "nm" means nanometers. "g" is gram. "Include" means that it may contain other steps as well as ingredients, including "by... Composition" and "mainly by... composition". The embodiments and the described aspects may be included or may be combined with those components, features, and other embodiments that are not explicitly disclosed, unless the non-compliance is explicitly stated. "In at least one embodiment" refers to one or more embodiments, or all or most of the embodiments have the features described.

The "viscosity" was measured at 25 ° C using a HAAKE Rotation Viscosimeter VT 550 using a cooling/heater and induction system. According to DIN 53019, the shear rate was 12.9 s <-1>. Water-soluble means that a solution which is judged to be clear by the naked eye can be formed in water at a concentration of 0.1% at 25 °C.

"Substantially free" means less than 1%, or less than 0.8%, or less than 0.5%, or less than 0.3%, or about 0% of the total ingredients or formulations.

"Keratin fiber" refers to a fiber composed of keratin. "Hair" refers to keratin fibers of mammals. In at least one embodiment, "hair" refers to the hair of a person. "Hair shaft" or "hair fiber" refers to a single line of hair that can be used interchangeably with the term "hair".

"Cosmetic acceptable" refers to the ingredients described which are in contact with human keratinous tissue without undue toxicity.

"Derivatives" include, but are not limited to, acetyl, ester, carboxylic acid, acid, salt, alcohol, and the like.

"Monomer" means a single, unpolymerized, chemical unit that can form macromolecules by polymerization under certain conditions.

"Kit" means a package comprising a plurality of components. "Kit" may be referred to as "kit-of-parts". An example of a kit is, for example, a first composition and a separately packaged second composition and optionally application instructions.

### Description of the invention

The present invention describes methods of strengthening keratin fibers. The method achieves a semi-permanent strengthening effect by using a fortified formula on the hair or by combining the formulation with a commercially available hair dye or bleaching ingredient. The method contains two different formulations, one of which has a pH greater than 7, and the other has a pH of less than 7. The first formula is alkaline and the second formula is acidic. The hair strengthening effect is maintained at least once after shampooing. Furthermore, the inventors have found that this method increases the water resistance and moisture resistance of the hair style and increases the ease of style and/or increases the manageability of the hair style after the shampoo is used. When mixed with commercially available hair dyes or bleaches, the alkaline hair strengthening formulation has no adverse effect on hair dyeing and bleaching.

The above effects are derived from the steps used, the order of these steps, and the specific ingredients including the active agent. The active ingredient in the acidic formulation diffuses into the hair shaft of the keratin fiber, reacts with the amino group in the keratin polypeptide, and crosslinks the functional groups in the keratin structure by providing sufficient cross-linking to resist instinctive recovery in keratin power. The acidic component cannot react with potential sulfhydryl groups in the hair because the reaction conditions are acidic. In theory, a potential sulfhydryl reaction requires deprotonation. Since the protonating agent in the present invention is an acid stronger than the sulfhydryl group, such a reaction cannot occur. The active ingredient in the alkaline formulation diffuses into the keratin fibers in the hair shaft, reacts with the carboxylic acid groups in the keratin polypeptide and crosslinks these functional groups. This results in a durable strengthening of keratin fibres, for example a durable hair damage repair.

Specific details of various aspects of the invention are set forth below.

### Cross-linking ingredients:

The crosslinking components of the present invention are different from each other and act synergistically. The order of use is not related to its effect, but when it is mixed with commercially available hair dyes or bleaches, the order of use is related to the effect. Commercially available hair dyes and bleaches contain a pH adjusting component that makes the pH alkaline. When the hair is oxidatively dyed, a change in pH of 0.5 causes a change in the final color of the hair. When used in commercially available hair bleaching formulations, a pH change of more than 0.5 will reduce bleaching. Therefore, when it is desired to add additives to commercially available hair dyes and bleaches, it is important that these additives do not alter the pH of the hair dye and bleach. Commercially available hair dyes and bleaches have a pH of from 8 to 12, so it is important to mix them with the alkaline ingredients of the present invention. When the formulation of the present invention is intended to be mixed with commercially available hair dyes and bleaches, the mixed formulation is used first, followed by the unmixed acidic hair strengthening composition. It is preferred to have a stay of between 1 and 45 minutes between steps. Optionally, the hair is washed and dried between the first step and the second step. Optionally, there is a waiting time of 1 to 45 minutes inbetween the application of the acidic and alkaline hair strengthening composition.

The acidic hair strengthening component (b) is generally used after the alkaline hair strengthening component (a), and contains at least bifunctional Bronsted acid as a crosslinking agent. At least bifunctional Bronsted acid is preferred for the acidic hair strengthening component because it binds to adjacent amino groups of the hair. Most bifunctional Bronsted-acids are naturally-derived which is preferred by consumers versus synthetic compounds. This is not only for health reasons, but also for sustainability and environmental reasons, as naturally obtained compounds decompose naturally and rapidly and do not require special treatment. And, they are relatively cheap.

The active agent in the alkaline hair strengthening component reacts with the carboxylic acid groups in the hair and provides further crosslinking in the hair protein. Hair contains keratin polypeptides containing a carboxylic acid group [-COOH], a hydroxyl group [-OH], an amino group [-NH₂], and potentially a sulfhydryl group [-SH]. Different active ingredients react with different functional groups. For example, at least bifunctional Bronsted acid reacts with an amino group, and at least a bifunctional proton acceptor reacts at the carboxyl group.

The weight concentration of at least bifunctional Bronsted acid and at least bifunctional proton acceptor to the total acidic or basic cross-linking component is from 1 to 30%, preferably from 2 to 25%, more preferably from 3 to 20%, It is particularly preferably 4 to 15%, and most preferably 5 to 10%. The molar ratio of at least bifunctional proton acceptor to at least bifunctional Bronsted acid is from 1:1 to 3:1, preferably from 1.5:1 to 2.5:1, particularly preferably from 2:1 to 2.5:1.

In at least one embodiment, the acidic and basic cross-linking components each comprise a cosmetically acceptable carrier. In at least one embodiment, the cosmetically acceptable carrier can be any such that the active ingredient can be dispensed into the cross-linking component to enable it to be applied to the hair. In at least one embodiment, the carrier is an aqueous carrier or an aqueous-alcoholic carrier. In at least one embodiment, when the carrier is an aqueous-alcoholic carrier, the carrier contains water and an alcohol. In at least one embodiment, the alcohol is selected from the group consisting of ethanol, isopropanol, propanol, or a mixture thereof. In at least one embodiment, when the carrier is an aqueous carrier, the carrier contains primarily water and is substantially free of alcohol. In at least one embodiment, the acidic and basic cross-linking components each independently comprise a safe and effective amount of a cosmetically acceptable carrier. In at least one embodiment, the acidic and basic crosslinking components each independently comprise from about 0.1% to 99%, or from about 1% to 98%, or from about 10% to 97%, or about 30% to about 95% water, by weight of the cross-linking component.

The acidic and basic cross-linking components may contain other ingredients. In at least one embodiment, the acidic and basic crosslinking components contain an antioxidant. Antioxidants have a role in providing long-term stability for the cross-linking component. In at least one embodiment, the acidic and basic cross-linking ingredients comprise from 0.001 % to 5%, or from 0.5% to 1.0%, of an antioxidant. In at least one embodiment, the antioxidant is selected from the group consisting of ascorbic acid (vitamin C), fatty acid ascorbate, ascorbic acid derivatives (eg, magnesium ascorbyl phosphate, sodium ascorbyl phosphate, ascorbyl sorbate), vitamin E, tocopherol sorbate, tocopheryl acetate, other esters of tocopherol, butyl hydroxybenzoic acid and its salts, peroxides, including hydrogen peroxide, perborates, thioglycolates, persulphates, 6-hydroxy- 2,5,7,8-tetramethylchroman-2-carboxylic acid (commercial name Trolox<TM>), gallic acid and its alkyl esters, especially propyl gallate, uric acid and its salts and alkyl ester, ferulic acid salt and alkyl ester, sorbic acid and its salt, lipoic acid, amine (for example, N-diethylhydroxylamine, aminoguanidine), sulfhydryl compounds (such as glutathione), dihydroxyl fumaric acid and its salts, lysine pidolate, arginine pidolate, nordihydroguaiaretic acid, bioflavonoids, curcumin, lysine, 1-methionine, proline, superoxide dismutase, silymarin, tea extract, grape skin or grape seed extract, melanin, rosemary extract and their mixtures. In at least one embodiment, the antioxidant is tocopherol sorbate or an ester of tocopherol. In at least one embodiment, the antioxidant is sodium benzoate. In at least one embodiment, the antioxidant is ferulic acid. The advantage of ferulic acid is that it enhances the oxidative stability of the formulation. In at least one embodiment, the acidic and basic cross-linking ingredients comprise a safe and effective amount of ferulic acid. In at least one embodiment, the crosslinking component contains from about 0.001% to 5%, or from about 0.5% to 1.0%, ferulic acid.

In at least one embodiment, the acidic and basic crosslinking components comprise a chelating agent. A chelating agent refers to an active material that can remove metal ions from the system by forming a complex with them, such that these metal ions cannot participate in or catalyze a chemical reaction. A chelating agent provides protection against UV radiation that can cause transitional flaking and skin damage and against other substances in the environment that can cause skin damage, by reducing the amount of local iron ions that can cause oxidation and pigmentation. The chelating agent can increase the long-term stability of the cross-linking component. In at least one embodiment, the acidic and basic crosslinking components comprise a safe and effective amount of a chelating agent. In at least one embodiment, the acidic and basic crosslinking component comprises a chelating agent selected from the group consisting of N-hydroxysuccinimide, EDTA, NTA, deferoxamine, hydroxamic acid and salts thereof, phytic acid, phytate, gluconic acid and salt, transferrin, lactoferrin, and mixtures thereof. In at least one embodiment, the acidic and basic crosslinking components comprise a safe and effective amount of a chelating agent. The acidic and basic cross-linking ingredients comprise from about 0.001% to 10%, or from about 0.01% to 5%, or from about 0.1% to 5%, or from about 0.5% to 1.0%, of a chelating agent. A chelating agent which can be used is disclosed in the patent document US Pat. No. 5,487,884, issued January 30, 1996, by Bissett et al., International patent application No. 91/16035, filed by Bush et al., published on October 31, 1991, and the International patent application Document No. 91/16034, by Bush et al., published on October 31, 1991. In at least one embodiment, the chelating agent is selected from the group consisting of N-hydroxysuccinimide, deferoxamine, hydroxamic acid and salts thereof, phytic acid, gluconic acid, and derivatives thereof, and mixture.

In at least one embodiment, the acidic and basic cross-linking ingredients are in a form suitable for use in hair. In at least one embodiment, the acidic and basic crosslinking component is an emulsion, a solution, or a dispersion. In at least one embodiment, the crosslinking component comprises a surfactant. The surfactant is capable of producing an emulsion. In at least one embodiment, the emulsion can be a water-in-oil emulsion, an oil-in-water emulsion, or a multiple emulsion. The advantage of the emulsion is that it is easy to use. The acidic and alkaline crosslinking components may be either a no-rinse or a rinse-off type. The acidic and basic cross-linking components may also be in the form of a hair conditioning composition.

The acidic and alkaline cross-linking component may also contain at least one cosmetic component selected from the group consisting of a hair styling polymer, conditioning agent, hair cleansing agent, or a mixture thereof. In at least one embodiment, the acidic and basic crosslinking components comprise a hair styling polymer. In at least one embodiment, the hair styling polymer is selected from the group consisting of nonionic hairstyling polymer, anionic hairstyling polymer, zwitterionic and/or amphoteric hairstyling polymer, cationic hair styling polymer, or mixtures thereof. The hair styling polymer that can be used can be found in the 2008 CTFA International Cosmetic Ingredient Dictionary Handbook, "Hair Styling Agents", version 12. Suitable hair styling polymers can be the polymers disclosed in European patent application No. 08151246.9, filed on 11 February 2008, from page 12 line 5 to page 19 line 1.

In at least one embodiment, the acidic and basic cross-linking component comprises from about 0.01% to about 10%, or from about 0.1% to about 8%, or from about 0.1% to about 5% by weight of the hair styling polymer.

In at least one embodiment, the crosslinking component comprises a non-ionic hair styling polymer. In at least one embodiment, the hair styling polymer is a natural or synthetic polymer. In at least one embodiment, the nonionic hair styling polymer is selected from the group consisting of polymers polymerized from the following monomers: vinyl pyrrolidone, vinyl caprolactam, vinyl ester, vinyl alcohol, vinyl acetate, (meth)acrylamide and/or a derivative thereof, (meth)acrylic acid, a salt thereof, and/or a derivative thereof, propylene, glycolic acid, crotonic acid, or a mixture thereof. For example, the commercial name for these polymers can be available under the trade names Luviskol^{®} or Luviset Clear^{®}.

In at least one embodiment, the crosslinking component comprises an anionic hairstyling polymer. In at least one embodiment, the anionic hair styling polymer is selected from the group consisting of: acrylic acid/alkyl acrylate/N-alkyl acrylamide terpolymer, vinyl acetate/crotonic acid copolymer, C1-C5-alkyl acrylate /(meth)acrylic acid copolymer, sodium polystyrene sulfonate, vinyl acetate/crotonic acid/vinyl alkanoate copolymer, vinyl acetate/crotonic acid/vinyl neodecanoate copolymer , aminomethylpropanol acrylic acid acrylate copolymer, vinylpyrrolidone / (meth)acrylic acid copolymer, methyl vinyl ether / maleic monoalkyl ester copolymer, aminomethyl propanolate of allyl methacrylate / Methacrylate copolymer, ethyl acrylate/methacrylic acid copolymer, vinyl acetate/mono-n-butyl maleate/isobornyl acrylate copolymer, octyl acrylamide/methacrylic acid copolymer, polyester of diethylene glycol, cyclohexanedimethanol, isophthalic acid and sulfoisophthalic acid, and mixtures thereof.

In at least one embodiment, the acidic and basic crosslinking components comprise a zwitterionic hair styling polymer. In at least one embodiment, the zwitterionic hair styling polymer is selected from the group consisting of: N-alkyl acrylamide / alkylaminoalkyl methacrylate / methacrylic acid copolymer, copolymers which are formed from at least one first monomer type which has quartenary amine groups, and at least one second monomer type which has acid groups, copolymers or fatty alcohol acrylates, of alkylamine oxide metyhcrylate and at least one monomer chosen from acrylic acid and methacrylic acid, methyacryloylethylbetaine/methacrylic acid and/or esters copolymers, polyquaternium-47, polyquaternium -43, oligomer or polymer of croton betaine or croton betain ester, and mixtures thereof.

In at least one embodiment, the acidic and basic crosslinking components comprise a cationic hair styling polymer. In at least one embodiment, the cationic hair styling polymer is a homopolymer or a copolymer in which a quaternary amine group is present in the polymer backbone or as a substituent on one or more of the cationic monomers . The ammonium group-containing monomer may be copolymerized with a non-cationic monomer. Suitable cationic monomers may be unsaturated free-radically polymerizable compounds which contain at least one cationic group, especially a vinyl ammonium salt, for example, a trialkyl methacryloxyalkyl ammonium, a trialkylacryloxyalkylammonium, a dialkyldiallylammonium and a quaternary vinylammonium with cyclic, cationic nitrogen-containing groups, such as pyridinium, imidazolium or quaternary pyrrolidones, e.g. alkylvinylimidazolium, alkylvinylpyridinium, or an alkyl vinyl pyrrolidone salt. The alkyl group in these monomers is preferably a low alkyl group such as a C1 to C7 alkyl group, and particularly preferably a C1 to C3 alkyl group. Suitable non-cationic monomers are selected from the group consisting of: methacrylamide and its derivatives, acrylates and derivatives thereof, vinyl caprolactone, vinyl caprolactam, vinyl pyrrolidone, vinyl ester, vinyl alcohol, propylene glycol, ethylene glycol. For example, suitable cationic hair styling polymers are polymers having the following trade names, Gafquat 755N, Gafquat 734, Gafquat HS 100, Luviquat HM 550, Merquat Plus 3300, Gaffix VC 713, Aquaflex SF 40. In at least one embodiment, the crosslinking component comprises a cationic hair styling polymer derived from a natural polymer. In at least one embodiment, the natural polymer is selected from the group consisting of: a cationic derivative of a polysaccharide, such as cellulose, starch, and or guar, chitosan, a salt thereof, and/or a derivative thereof, or a mixture thereof. In at least one embodiment, the cationic hair styling polymer is selected from the group consisting of: polyquaternium-4, polyquaternium-10, polyquaternium-24, guar hydroxypropyltrimonium chloride, and mixture.

In at least one embodiment, the acidic and alkaline cross-linking ingredients comprise a hair conditioning component. The acidic and alkaline cross-linking ingredients comprise any suitable, conventional hair conditioning ingredient. The hair conditioning ingredient here refers to any cosmetic acceptable ingredient, which can improve the appearance of the hair, such as making the hair shiny, easy to comb, good touch, volume. The hair conditioning ingredients that can be used can be found in the 2008 CTFA International Cosmetic Ingredient Dictionary Handbook, "Hair Conditioning Agents", version 12. In at least one embodiment, the hair conditioning component is selected from the group consisting of: a cationic surfactant, a nonionic surfactant, a silicon compound, an organic oily conditioning ingredient, and mixtures thereof. Suitable hair conditioning ingredients can be the polymers disclosed in European patent application No. 08151246.9, filed on 11 February 2008, from page 19 line 3 to page 27 line 33.

In at least one embodiment, the conditioning component is a cationic surfactant. In at least one embodiment, the cationic surfactant contains an amino or quaternary ammonium moiety. In at least one embodiment, the acidic and basic crosslinking components comprise from about 0.05% to 3.5%, or from about 0.1% to 3.0%, or from about 0.5% to 2.5%, of a cationic surfactant. In at least one embodiment, the cationic surfactant has the formula II: 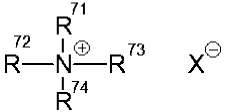 wherein at least one of R⁷¹, R⁷², R⁷³ and R⁷⁴ is selected from: an aliphatic group of from 8 to 30 carbon atoms; an aromatic, alkoxy, polyoxyalkylene, alkylamido, hydroxyalkyl, aryl; or an alkylaryl group having from 7 to 22 carbon atoms; wherein the remainder of R⁷¹, R⁷², R⁷³ and R⁷⁴ are independently selected from the group consisting of: an aliphatic group consisting of from 1 to 22 carbon atoms; and an aromatic, alkoxy, polyoxyalkylene, alkylamido, hydroxyalkyl, aryl or alkylaryl group having up to 22 carbon atoms; wherein X is selected from the group consisting of: halogen, acetate, citrate, lactate, glycolate, phosphate, nitrate, sulfonate, sulfate, alkylsulfate, alkyl sulfonate radicals, and mixtures thereof. In at least one embodiment, cationic surfactant is according to Formula II (see above), wherein at least one of R⁷¹, R⁷², R⁷³ and R⁷⁴ is an aliphatic group having from 16 to 24 carbon atoms; wherein the remainder of R⁷¹, R⁷², R⁷³ and R⁷⁴ are independently selected from the group consisting of aliphatic groups having from 1 to 4 carbon atoms; wherein X is selected from the group consisting of: chloride or sulfate. In at least one embodiment, the cationic surfactant is selected from the group consisting of: behenyltrimethylammonium chloride, methyl sulfate or ethyl sulfate; stearyltrimethylammonium chloride, methyl sulfate or ethyl sulfate; and mixtures thereof. It is believed that a longer alkyl group provides improved smoothness and soft feeling on wet and dry hair, compared to cationic surfactants with a shorter alkyl group. It is also believed that such cationic surfactants can provide reduced scalp irritation, compared to those having a shorter alkyl group. In at least one embodiment, the cationic surfactant is a di-long alkyl quaternized ammonium salt selected from the group consisting of: dialkyl (14-18 carbons) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, and mixtures thereof. In at least one embodiment, the cationic surfactant is a tertiary amidoamine having an alkyl group of from about 12 to about 22 carbons. In at least one embodiment, the cationic surfactant is selected from the group consisting of: cetyl trimethyl ammonium salts; behenyl trimethyl ammonium salts; dimethyl ditallow ammonium salts; stearyl amidopropyl dimethylamine; (di)esterquats; quaternium 8, 14, 15, 18, 22, 24, 26, 27, 30, 33, 37, 53, 60, 61, 72, 78, 80, 81, 82, 83, 84, and/or 91; or mixtures thereof.

In at least one embodiment, the conditioning agent is a non-ionic surfactant. Suitable non-ionic surfactants may be surfactants having a HLB of less than 8. Suitable nonionic surfactants may be selected from glyceryl esters; sugar esters; alkylpolyglucoside ethers; oleyl- or isostearylpolyglucoside; polyoxyethylene (20) sorbitan monostearate; or mixtures thereof.

In at least one embodiment, the conditioning agent is a silicone compound. In at least one embodiment, the silicone compound is volatile or nonvolatile, and/or soluble or insoluble silicones. For example, suitable silicone conditioning agents are available under the tradenames SF 1075 methyl phenyl fluid (Electric company); DC200 Fluid, DC244, DC245, DC345, Dow 5-7113, DC556 Cosmetic Grade Fluid, DC1248 (Dow Corning). In at least one embodiment, the acidic as well as the caustic crosslinking composition comprises a conditioning agent being the reaction product of: (a) an aminosilane; (b); polysiloxane; and optionally (c) a polyether. In at least one embodiment, the acidic as well as the caustic crosslinking composition comprises a conditioning agent being the reaction product of: (a) an aminosilane; (b); polysiloxane; and (c) a polyether. In at least one embodiment, the acidic as well as the caustic crosslinking composition comprises a conditioning agent, and wherein the conditioning agent is selected from the group consisting of: epoxyaminosilane copolymers, and polysiloxane/polyurea block copolymers, and mixtures thereof. In at least one embodiment, the acidic as well as the caustic crosslinking composition comprises a conditioning agent being the reaction product of: (a) an aminosilane; (b) polysiloxane; and (c) a polyether; and optionally (d) an amine. In at least one embodiment, the polysiloxane is an epoxy encapped polysiloxane. In at least one embodiment, the polysiloxane comprises at least two oxirane or oxetane groups. In at least one embodiment, the polysiloxane comprises from about 10 to about 450 silicon atoms, or from about 40 to about 400 silicon atoms, from about 75 to about 350 silicon atoms, from about 150 to about 250 silicon atoms. In at least one embodiment, the polysiloxane is an epoxy encapped polysiloxane. In at least one embodiment, the polyether has the structure CH₂(O)CHCH₂O(CH₂(CH₃)CH₂O)ₙCH₂CH(O)CH₂ (average) wherein n is an integer from 1 to 10. In at least one embodiment, the amine comprises from 1 to 10 carbon atoms, or from 2 to 5 carbon atoms. In at least one embodiment, the amine is an alkylamine that is substituted with at least one alkyl group. In at least one embodiment, the amine is selected from the group consisting of: methylamine, ethylamine, propylamine, ethanol amine, isopropylamine, butylamine, isobutylamine, hexylamine, dodecylamine, oleylamine, aniline aminopropyltrimethylsilane, aminopropyltriethylsilane, aminomorpholine, aminopropyldiethylamine benzylamine, napthylamine 3-amino-9-ethylcarbazole, 1-aminoheptaphlorohexane, 2,2,3,3,4,4,5,5,6,6,7,7,8,8,8-pentadecafluoro-1-octanamine, and mixtures thereof. In at least one embodiment, the amine is selected from the group consisting of: methylethylamine, methylhexylamine, methyloctadecylamine, diethanolamine, dibenzylamine, dihexylamine dicyclohexylamine, piperidine, pyrrolidine phthalimide, and mixtures thereof. In at least one embodiment, the conditioning agent is an epoxyaminosilane copolymer. In at least one embodiment, the conditioning agent is conditioning agent being the reaction product of: (a) an aminosilane; (b) polysiloxane, wherein the polysiloxane comprises from about 10 to about 450 silicon atoms, or from about 40 to about 400 silicon atoms; and (c) a polyether; and (d) an amine, wherein the amine is an alkylamine that is substituted with at least one alkyl group.

In at least one embodiment, the acidic as well as the caustic conditioning agent is selected from the group consisting of: epoxyaminosilane copolymers, and polysiloxane/polyurea block copolymers, and mixtures thereof. In at least one embodiment, the conditioning agent is a polydimethylsiloxane-derivative comprising aminoalkyl groups and having an amine number of at least 0.1 meq/g of polydimethylsiloxane. Such polydimethylsiloxane-derivative can, for example, be methoxy-terminated or hydroxy-terminated, or mixtures thereof.

In at least one embodiment, the conditioning agent is an organic oily conditioning agent. In at least one embodiment, the organic oily conditioning agent is nonvolatile, water-insoluble, oily or fatty. Organic oily conditioning agents may be selected from hydrocarbon oils and fatty esters. In at least one embodiment, the conditioning agent is a fatty alcohol. In at least one embodiment, the fatty alcohol is a non-volatile low melting point fatty alcohol. In at least one embodiment, the conditioning agent is a fatty alcohol and the fatty alcohol is selected from the group consisting of: capryl alcohol, lauryl alcohol, stearyl alcohol, cetyl alcohol, myristyl alcohol, palmitoleyl alcohol, and mixtures thereof.

The acidic as well as the caustic crosslinking composition may further comprise at least one direct hair dye. In at least one embodiment, the crosslinking composition comprises from about 0.01% to about 15%, or from about 0.1% to about 10 %, or from about 0.5% to about 8% direct hair dye.

The acidic as well as the caustic crosslinking composition may further comprise at least one viscosity-modifying agent. In at least one embodiment, the crosslinking composition comprises from about 0.01% to about 20%, or from about 0.05% to about 10%, or from about 0.1% to about 5% viscosity-modifying agent.

The acidic as well as the caustic crosslinking composition may further comprise at least one emulsifier and/or surfactant. In at least one embodiment, the emulsifier and/or surfactant is selected from nonionic surfactants; anionic surfactants; amphoretic surfactants; or mixtures thereof. In at least one embodiment, the acidic as well as the caustic crosslinking composition comprises from about 0.01% to about 20%, or from about 0.05% to about 10%, or from about 0.1% to about 5%, emulsifier and/or surfactant.

The acidic as well as the caustic crosslinking composition may further comprise at least one pigment. In at least one embodiment, the pigment is selected from natural pigments; synthetic pigments; or mixtures thereof. The pigments may be selected from organic pigment, inorganic pigment; or mixtures thereof. The pigments may be selected from coloured pigments; pearlescent pigments; or mixtures thereof. Said acidic as well as the caustic crosslinking composition may comprise from about 0.01% to 10%, or from about 1% to about 2% pigment present in the product mass in undissolved form by weight of the total acidic as well as the caustic crosslinking composition. The acidic as well as the caustic crosslinking composition may comprise pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water-soluble components such as those having C.I. Names.

In at least one embodiment, the acidic as well as the caustic crosslinking composition comprises at least one particulate substance. In at least one embodiment, the particulate substance is selected from silica; silicates; aluminates; clay earths; mica; insoluble salts, particularly insoluble inorganic metal salts; metal oxides; minerals; insoluble polymer particles; or mixtures thereof. In at least one embodiment, the acidic as well as the caustic crosslinking composition comprises from about 0.01% to about 10%, or from about 0.05% to about 5% of at least one particulate substance. In at least one embodiment, the acidic as well as the caustic crosslinking composition is substantially free of a particulate substance such as clay.

In at least one embodiment, the acidic as well as the caustic crosslinking composition comprises at least one preservative. In at least one embodiment, the acidic as well as the caustic crosslinking composition may comprise from about 0.01% to about 5% by weight, or from about 0.05% to about 1% preservative.

A variety of additional optional ingredients may be incorporated into the acidic as well as the caustic crosslinking composition of the present invention. Non-limiting examples of these additional ingredients may be selected from preservatives; antioxidants; sequestering agents; solvents; fragrances & perfumes; fillers; screening agents; odour absorbers; colouring materials; lipid vesicles; detersive surfactants; thickening agents and suspending agents; viscosity modifiers; pearlescent aids; UV-filters and sunscreens; agents for combating free radicals; polyvinyl alcohol; pH adjusting agents; salts; colouring agents; polymer plasticizing agents; direct dyes; or mixtures thereof. The acidic as well as the caustic crosslinking composition may comprise from about 0%, or from about 0.1% to about 5% antimicrobial agents. In at least one embodiment, the acidic as well as the caustic crosslinking composition comprises an organic acid selected from the group consisting of: glycine, L-methionine, L-arginine, biotin, creatine, and mixtures thereof. In at least one embodiment, the acidic as well as the caustic crosslinking composition comprises panthenol. In at least one embodiment, the acidic as well as the caustic crosslinking composition comprises a wax compound. In at least one embodiment, the acidic as well as the caustic crosslinking composition comprises beeswax.

In at least one embodiment, the acidic as well as the caustic crosslinking composition has a viscosity, measured at 25°C, of from about 0.1 mPa·s to about 1,000,000 mPas, or from about 1 mPa·s to about 80,000 mPa·s, or from about 5 mPa·s to about 3,500 mPa·s. The viscosity is measured by HAAKE Rotation Viscometer VT 550 with cooling/heating vessel and sensor systems according to DIN 53019 (MV-DIN, SV-DIN), shear rate is 12.9 s⁻¹.

In at least one embodiment, the acidic as well as the caustic crosslinking composition comprises a buffering agent. In at least one embodiment, the buffering agent is a phosphate buffer. In at least one embodiment, the buffering agent is a borate buffer or a carbonate buffer. In at least one embodiment, the buffering agent is selected from the group consisting of: glycine/sodium hydroxide; sodium carbonate/sodium hydrogen carbonate, sodium tetraborate/sodium hydroxide; sodium bicarbonate/sodium hydroxide; ammonium chloride/ammonia. The buffering agent has the advantage of controlling the pH, which aids the stability of the crosslinking composition. In at least one embodiment, the acidic as well as the caustic crosslinking composition comprises an alkalizing agent and/or an agent for adjusting the pH value. The acidic as well as the caustic crosslinking composition may further comprise a protonating agent. The protonating agent may be a monoprotic or polyprotic acid, water-soluble or water-insoluble acid, and/or an organic or inorganic acid. In at least one embodiment, the protonating agent is selected from formic acid, acetic acid, sulfuric acid, hydrochloric acid, citric acid, and mixtures thereof.

### Method

Described herein is a hair strengthening and/or hair repairing method comprising: (a) applying a caustic crosslinking composition to hair and leaving the caustic hair strengthening composition on for 1 to 45 minutes; (b) optionally rinsing, shampoo'ing and/or drying the hair drying; (c) applying an acidic crosslinking composition to hair and leaving the acidic hair strengthening composition on for 1 to 45 minutes; (d) optionally rinsing, shampoo'ing and/or drying the hair. Prior to step (a) the hair strengthening composition can be mixed with commercially available hair colouring or bleaching formulations. If mixed with commercially available hair colouring or bleaching formulations the method steps are in the order (a) then (b) then (c) then (d). If not mixed with commercially available hair colouring or bleaching formulation the method steps can be (c) then (d) then (a) than (b). In the method of the present invention, the acidic as well as the caustic crosslinking composition may be applied on wet hair and/or on dry hair.

In an embodiment, prior to the first step of the method which can either be step (a) or step (c) the hair is washed with a shampoo, for example a cleansing shampoo. In an embodiment, at the end of the treatment cycle, thus following either step (b) or step (d), depending on if the method started with step (a) or step (c), the hair is conditioned with a conditioning formulation comprising a conditioning agent. Conditioning agents are disclosed herein and are suitable for this embodiment. In an embodiment, the hair is dried using a blow dryer and a brush.

In an embodiment, the method relates to a hair strengthening and/or hair repairing method comprising: (a) applying a caustic hair care composition to hair, wherein the hair care composition comprises an at least bi-functional Brønsted-base as defined in claim 1 and of the general formula X-R-Y, wherein X and Y are proton-acceptor groups and R is an organic spacer comprising 1 to 20 carbon atoms, and 0 to 5 oxygen atoms, and 0 to 5 nitrogen atoms, and X-R-Y having a molecular weight of less than 500 g/mol, and leaving the caustic hair strengthening composition on for 1 to 45 minutes, (b) optionally rinsing or drying the hair, (c) applying to the hair an acidic formula comprising a crosslinking composition comprising an at least bi-functional organic acid as defined in claim 1 and capable of reacting with the amine groups of the hair, and leaving the acidic hair strengthening composition on for 1 to 45 minutes, (d) optionally rinsing, shampoo'ing and/or drying the hair, characterized in that the composition of step (a) has a pH 7 to 12 and is alkaline and has a pH greater than 7, and the composition of step (c) has a pH of 1.5 to 7 and is acidic and has a pH of less than 7. Optionally, the hair strengthening composition of step (a) can be mixed into commercially available hair colouring or hair bleaching formulations prior to the application. When applied without prior mixing into either a commercially available hair colouring or bleaching formulation the hair strengthening method can be altered such that the hair strengthening composition of step (c) is applied before hair strengthening composition of step (a) is.

### Applying a hair care composition to hair

The present invention relates to a hair strengthening and/or repairing method comprising: (a) applying a caustic hair care composition to hair, wherein the hair care composition comprises an at least bi-functional Brønsted-base as defined in claim 1 and of the general formula X-R-Y, wherein X and Y are proton-acceptor groups and R is an organic spacer comprising 1 to 20 carbon atoms, and 0 to 5 oxygen atoms, and 0 to 5 nitrogen atoms, and X-R-Y having a molecular weight of less than 500 g/mo, and (c) applying to the hair an acidic formula comprising a crosslinking composition comprising an at least bi-functional organic acid as defined in claim 1 and capable of reacting with the amine groups of the hair, involving applying onto hair from about 0.01 gram to about 5 gram of said compositions per gram hair. In an embodiment, the acidic as well as the caustic composition is on the hair for at least 1 min, or from about 5 min to about 45 min, or from about 10 min to about 40 min, or from about 20 min to about 35 min, prior to carrying out the remaining step, either (a) or (c), depending on if the method was started with step (a) or (c).

### Hair drying

The hair straightening and/or hair relaxing method may optionally comprise hair drying in steps (b) and/or (d). In an embodiment, the hair drying is carried out by a blow drier. In an embodiment, the hair drying is carried out for a duration of from about 1 min to about 45 min, or from about 2 min to 20 min, or from about 5 min to 15 min. In general, following the hair drying, the hair can still be damp, but needs to have reasonable e.g. 75% hair fibre separation of the head of hair. Some residual moisture in the hair is acceptable. In an embodiment, the hair drying is carried out by a hood appliance. In an embodiment, the hair drying is carried out by towelling hair and/or by pressing hair with hands.

Hair dryer or blow dryer distances between device and head are typically down to about 10 cm. Blow dryers direct hot air through some sort of attachment for combing or otherwise treating the hair. A blow dryer is typically used such that the distance to the hair (for example at a distance of 20 or 30 or 40 centimetres) and often is used with the aid of a comb or a brush. In an embodiment, the hair drying is carried out by a blow drier at a temperature of from about from 50°C to about 100°C. In an embodiment, the hair drying is carried out by a blow drier at a temperature of up to 130°C. In an embodiment, the hair drying is carried out with a blow drier with brushing to help styling the hair.

In an embodiment, the hair strengthening and/or repairing method comprises in addition to to steps (a), (b), (c), (d) also a hair straightening step (e). The hair straightening step (e) comprises using a hair straightening appliance comprising metal or ceramic plates. In an embodiment, the metal or ceramic plates are provided to a temperature of from about 100°C to about 280°C. In an embodiment, the metal or ceramic plates are provided to a temperature of from about 110°C to about 250°C, or from about 120°C to about 240°C, or from about 140°C to about 230°C, or from about 160°C to about 220°C, or from about 180°C to about 210°C, or from about 190°C to about 200°C.

In an embodiment, the 'straightening of the hair with the appliance' is carried out for a duration of from about 1 min to about 45 min, or from about 2 min to 20 min, or from about 5 min to 15 min. In an embodiment, the 'mechanically straightening the hair with the appliance' is carried out for a duration of for at least 10 min, or for at least 12 min.

In an embodiment, method (a) to (d) is repeated from 2 to 4 times per month on an ongoing basis for the purpose of strengthening hair and reducing hair damage. In an embodiment, the caustic crosslinking composition may comprise a first, second, and third crosslinking agent. The first crosslinking agent may be 4,7,10-Trioxa-1,13-tridecanediamine, the second may be 4,9-Dioxa-1,12-dodecanediamine, and the third crosslinking agent may be 1, 11-Diamino-3,6,9-trioxaundecane.

In an embodiment, the crosslinking composition may comprise:
- from about 3% to about 24% 4,7,10-Trioxa-1,13-tridecanediamine, 4,9-Dioxa-1,12-dodecanediamine, and 1,11-Diamino-3,6,9-trioxaundecane, present at a weight ratio of 1:1:1, 2:1:1, or 4:1:1
- optionally, a buffering agent;
- a cosmetically acceptable carrier;
- a conditioning agent being the reaction product of: (a) an aminosilane; (b); polysiloxane; and optionally (c) a polyether;
and wherein the composition has a pH of from about pH 7 to about pH 12, and is alkaline and has a pH greater than 7.

In an embodiment, the formulation comprises from about 0.1% to about 15%, or from about 1% to about 10%, or from about 2% to about 5% conditioning agent being the reaction product of: (a) an aminosilane; (b); polysiloxane; and optionally (c) a polyether. In an embodiment, the conditioning agent is the reaction product of: (a) an aminosilane; (b); polysiloxane; (c) a polyether.

In an embodiment, a kit may comprise: (i) a caustic crosslinking composition; (ii) an acidic crosslinking composition; (iii) a conditioning composition. In an embodiment, the kit may be for strengthening and repairing damaged hair. In an embodiment, the kit may be for improving ease of hair styling.

In an embodiment, the crosslinking agent may be used for strengthening hair and /or repairing damaged hair. In an embodiment, the caustic as well as the acidic crosslinking composition may be used for improving ease of styling of the hair.

### EXAMPLES

The following examples further describe and demonstrate embodiments within the scope of the present invention. The examples are given solely for the purpose of illustration, and are not to be construed as limitations of the present invention since many variations thereof are possible without departing from its scope.

### Example compositions for the caustic crosslinking composition (total mass 100q):

Liquid A: 10 g 4,7,10-Trioxa-1,13-tridecanediamine, 5 g 4,9-Dioxa-1,12-dodecanediamine, 5 g 1,11-Diamino-3,6,9-trioxaundecane, 2 g epoxyaminosilane copolymer, QSP water
Liquid B: 25 g 4,7,10-Trioxa-1,13-tridecanediamine, QSP water
Liquid C: 12 g 4,7,10-Trioxa-1,13-tridecanediamine, 3 g 4,9-Dioxa-1,12-dodecanediamine, 3 g 1,11-Diamino-3,6,9-trioxaundecane, 2 g epoxyaminosilane copolymer, QSP water
Rinse-out conditioner A: 16 g 4,7,10-Trioxa-1,13-tridecanediamine, 8 g 4,9-Dioxa-1,12-dodecanediamine, 1.00 g cetyltrimethyl ammonium chloride, 1.00 g polymethylphenyl siloxane, 0.40 g phenoxy ethanol, 0.20 g PHB-methylester, 1.00 g Dow Corning 949 Cationic Emulsion^{®}, 5.00 g isododecane, 0.40 g perfume oil, QSP water.
Rinse-out Conditioner B: 18g 4,7,10-Trioxa-1,13-tridecanediamine, 1.00 g cetyltrimethyl ammonium chloride, 1.00 g polymethylphenyl siloxane, 0.40 g phenoxyethanol, 0.20 g PHB-methylester, 8.00 g Dow Corning 57113 Cationic Emulsion^{®}, 5.00 g isododecane, 0.40 g perfume oil, QSP water.
Leave-in Conditioner A: 6 g 4,7,10-Trioxa-1,13-tridecanediamine, 3 g 4,9-Dioxa-1,12-dodecanediamine,, 1.00 g cetyltrimethyl ammonium chloride, 1.00 g polymethylphenyl siloxane, 0.40 g phenoxy ethanol, 0.20 g PHB-methylester, 8.00 g Momentive^{™} Cationic Emulsion^{®}, 5.00 g isododecane, 0.40 g perfume oil, QSP water.
Leave-in Conditioner B: 10 g 4,7,10-Trioxa-1,13-tridecanediamine, 5 g 1,11-Diamino-3,6,9-trioxaundecane,, 0.10 g vitamin E-acetate, 0.50 g polymethylphenyl siloxane, 10.00 g propylene glycol, 0.50 g behenyl trimethylammonium chloride, 0.05 g sodium chloride, 0.30 g d-panthenol, 0.30 g PHB-propylester, 2.00 g isododecane, 0.20 g perfume oil, QSP water.

### Example compositions for the acidic crosslinking composition (total mass 100q):

Liquid A': 25 g Maleic Acid, QSP water
Liquid B': 25 g Itaconic Acid, QSP water
Liquid C': 12 g Maleic Acid, 12 g Itaconic Acid, 3 g 1,11-Diamino-3,6,9-trioxaundecane, 2 g epoxyaminosilane copolymer, QSP water
Rinse-out conditioner A': 12 g Maleic Acid, 1.00 g cetyltrimethyl ammonium chloride, 1.00 g polymethylphenyl siloxane, 0.40 g phenoxy ethanol, 0.20 g PHB-methylester, 1.00 g Dow Corning 949 Cationic Emulsion^{®}, 5.00 g isododecane, 0.40 g perfume oil, QSP water.
Rinse-out Conditioner B': 6 g Maleic Acid, 6 g Itaconic Acid, 1.00 g cetyltrimethyl ammonium chloride, 1.00 g polymethylphenyl siloxane, 0.40 g phenoxyethanol, 0.20 g PHB-methylester, 8.00 g Dow Corning 57113 Cationic Emulsion^{®}, 5.00 g isododecane, 0.40 g perfume oil, QSP water.
Leave-in Conditioner A': 3 g Maleic Acid, 1.00 g cetyltrimethyl ammonium chloride, 1.00 g polymethylphenyl siloxane, 0.40 g phenoxy ethanol, 0.20 g PHB-methylester, 8.00 g Momentive^{™} Cationic Emulsion^{®}, 5.00 g isododecane, 0.40 g perfume oil, QSP water.
Leave-in Conditioner B': 3 g Maleic Acid, 3 g Itaconic Acid, 0.10 g vitamin E-acetate, 0.50 g polymethylphenyl siloxane, 10.00 g propylene glycol, 0.50 g behenyl trimethylammonium chloride, 0.05 g sodium chloride, 0.30 g d-panthenol, 0.30 g PHB-propylester, 2.00 g isododecane, 0.20 g perfume oil, QSP water.

### Data

The hair strengthening efficacy is tested for the caustic as well as the acidic crosslinking compositions of the present invention. Switches of low lift natural hair are employed. These are shampooed with a K-PAK clarifying shampoo to ensure the hair is in a clean state with no residues that could affect the end result. The switches are then rinsed. Excess water is removed from the hair by wringing out the switches. The switches are treated with a crosslinking composition which comprises active agents as listed in TABLE 1 and QSP water buffered at pH 10 for the caustic crosslinking composition and QSP water buffered at pH 4 for the acidic crosslinking compositions. These ingredients are mixed on a spinner plate for 15 mins. 0.5 g of crosslinking composition per 1 g hair is employed. The crosslinking composition is left on the hair for 30 minutes. After this time, the hair is blow dried and brushed with a standard metal comb for 500 strokes. Hair strengthening and hair damage is assest via recording of the weight of broken hair fibers collected from the combing and normalized to the weight of the hair switches. 5 hair switches per experiment are treated and combed, results are averaged. When a first and a second treatment were employed, hair switches were first treated with the caustic hair strengthening composition according to the above mentioned description without thehair drying step, then followed by the treatment of the acidic hair strengthening composition as per the above mentioned descripten, including hair drying.

**Table 1. Hair breakage results after n combing strokes**

| Treatment | 50 strokes | 250 strokes | 500 strokes |
|---|---|---|---|
| Reference: Untreated hair | 5,0% | 8,1% | 9,7% |
| 25% Maleic Acid | 2,8% | 4,1% | 6,3% |
| 25% Itaconic Acid | 2,5% | 3,9% | 6,4% |
| 12% Maleic Acid + 12% Itaconic Acid | 2,5% | 4,4% | 5,9% |
| 15% 4,7,10-Trioxa-1,13-tridecanediamine | 2,9% | 4,4% | 6,8% |
| 15% 4,9-Dioxa-1,12-dodecanediamine | 3,1% | 3,9% | 5,9% |
| 15 % 1, 11-Diamino-3,6,9-trioxaundecane | 3,1% | 4,2% | 6,2% |
| First: 25% Maleic Acid | 0,8% | 2,1% | 3,3% |
| Second: 15% 4,7,10-Trioxa-1,13-tridecanediamine | | | |
| First: 25% Maleic Acid | 1,1% | 2,3% | 3,2% |
| Second: 15% 4,9-Dioxa-1,12-dodecanediamine | | | |
| First: 25% Maleic Acid | 1,0% | 1,9% | 3,4% |
| Second: 1,11-Diamino-3,6,9-trioxaundecane | | | |
| First: 25% Itaconic Acid | 1,2% | 2,5% | 3,1% |
| Second: 5% 4,7,10-Trioxa-1,13-tridecanediamine | | | |
| + 5% 4,9-Dioxa-1,12-dodecanediamine | | | |
| + 5% 1, 11-Diamino-3,6,9-trioxaundecane | | | |

When averaging over five experimental results, the relative standard deviation is less than 15%. As can be seen in Table 1, the sequential application of a caustic hair strengthening composition of the present invention with an acidic hair strengthening composition of the present invention significantly reduces hair breakage. Same hair breakage reduction results were achieved when the caustic hair strengthening composition was first mixed into a commercially available hair coloring formulation, left on the hair switch for a time specified by the hair color manufacturer's guidance, followed by applying the acidic hair strengthening composition as per instructions above, followed by subsequent hair drying.

To color human hair using oxidative dye technology it is generally necessary to treat the hair with a mixture of suitable oxidative coloring agents and at least one dye oxidising agent. Hydrogen peroxide is the most commonly used dye oxidising agent. However, in addition to dye oxidation, hydrogen peroxide treatment of the hair can also solubilise the colored melanin component in the hair and can lead to undesirable hair qualities, such as poor condition, due to increased brittleness and hair damage. These undesirable qualities are in part due to the necessary conditions of conventional peroxide treatment, as part of the hair coloring process, which requires high pH (>pH 9), extended exposure (from 10 to 60 minutes) and relatively high concentration of oxidising solutions (up to 20% volume of oxygen) in order to deliver effective dye oxidisation. Thus there is a need for hair coloring compositions which can oxidise dyes and color the hair effectively and, at the same time, strengthen the hair to provided prevent hair damage.

The process for hair bleaching is very similar to the process of hair colouring. Bleaching, basically, is a process of removing the natural color from hair. Because of the virtually unlimited variations of hair colors, bleaching per so, does not usually produce a uniform or aesthetically pleasing color in 'hair, nor will it produce a color tone other than that inherent in the hair. For these reasons hair that has been bleached is subsequently treated with a hair toner, a composition containing a hair dye which imparts the desired end-color to the bleached hair. The degree to which the natural color must be bleached from the hair is primarily determined by the desired endcolor. The toners do not lighten the shade of hair to any great extent; they impart their tone coloration to hair pre-bleached to the basic blonde shade desired, e.g. pastel blonde color tone is achieved in hair probleached to pale blonde not in hair pre-bleached only to a light brown.

Applied by itself or mixed with commercially available hair colouring or hair bleaching formulations, followed by subsequent application of the acidic hair strengthening composition, treating hair with the caustic as well as the acidic hair strengthening composition in a method of the present invention improves the quality of the hair reduces hair breakage, reduces hair damage, improves luster and hair shine, eases hair styling and improves moisture resistance of the hair.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "10%" is intended to mean "about 10%".

The citation of any document is not an admission that it is prior art with respect to any document disclosed or claimed herein or that it alone, or in any combination with any other reference or references, teaches, suggests or discloses any such embodiment. While particular embodiments have been illustrated and described herein, it should be understood that various other changes and modifications may be made without departing from the scope of the claimed subject matter. Moreover, although various aspects of the claimed subject matter have been described herein, such aspects need not be utilized in combination. It is therefore intended that the appended claims cover all such changes and modifications that are within the scope of the claimed subject matter.

## Claims

1. A method for strengthening hair and reducing hair breakage, comprising the steps of:
a) applying to the hair a first formulation, the first formulation comprising an at least bi-functional Brønsted-base of the formula X-R-Y, and
b) applying to the hair a second formulation, the second formulation comprising an at least bi-functional organic acid capable of reacting with the amine groups of the hair,
**characterized in that**
• the at least bi-functional Brønsted-base of the formula X-R-Y in the first formulation is 4,7,10-trioxa-1,13-tridecanediamine or a mixture of 4,7,10-trioxa-1,13-tridecanediamine and 4,9-dioxa-1,12-dodecanediamine or a mixture of 4,7,10-trioxa-1,13-tridecanediamine and 1,11-diamino-3,6,9-trioxaundecane or a mixture of 4,7,10-trioxa-1,13-tridecanediamine, 4,9-dioxa-1,12-dodecanediamine and 1,11-diamino-3,6,9-trioxaundecane, and
• the at least bi-functional organic acid in the second formulation is maleic acid or itaconic acid or a mixture of maleic acid and itaconic acid, and
• the first formulation (a) has a pH of 7 to 12, and the second formulation (b) has a pH of 1.5 to 7, and
• the first formulation (a) is alkaline and has a pH greater than 7, and the second formulation (b) is acidic and has a pH of less than 7.

2. The method of claim 1, wherein the concentration of the at least bi-functional Brønsted-base in step (a) is 1 - 30% by weight.

3. The method of claim 1, wherein the concentration of the at least bi-functional organic acid in step (b) is 1 - 30% by weight.

4. The method of claims 1, 2, 3 wherein step (a) is performed prior to step (b).

5. The method of claims 1, 2, 3 wherein step (b) is performed prior to step (a).

6. The method of claims 1 to 3, wherein the hair is dried inbetween steps (a) and (b) and the drying time is 1 to 60 minutes.

7. The method of claim 6, wherein a drying device is used for drying the hair.

8. The method of claims 1 to 3, wherein the formulation of step (a) is left on the hair for 1 to 45 minutes.

9. The method of claims 1 to 3, wherein the formulation of step (b) is left on the hair for 1 to 45 minutes.

10. The method of claims 1 to 3, wherein there is a waiting time inbetween the applications of steps (a) and (b) or (b) and (a) of 1 to 60 minutes.

11. The method of claims 1 to 3, wherein the formulations of steps (a) and (b) are separately mixed into a cosmetically acceptable carrier and where the cosmetically acceptable carrier of the formulation of step (a) is either identical or not to the cosmetically acceptable carrier of the formulation of step (b).

12. The method of claims 1 to 3, wherein the formulation of step (a) is mixed into a commercially available hair colouring or hair bleaching formulation prior to the application to hair.

13. The method of claims 1 to 3, wherein prior to the application of step (a) the hair is treated with a thioglycolic acid containing hair care composition.

14. A kit for the strengthening of hair comprising at least two separate formulations (a) and (b), wherein the first formulation (a) comprises an at least bi-functional Brønstedbase of the general formula X-R-Y, and wherein the second formulation (b) comprises an at least bi-functional organic acid capable of reacting with the amine groups of the hair, and wherein
• the at least bi-functional Brønsted-base of the formula X-R-Y in the first formulation is 4,7,10-trioxa-1,13-tridecanediamine or a mixture of 4,7,10-trioxa-1,13-tridecanediamine and 4,9-dioxa-1,12-dodecanediamine or a mixture of 4,7,10-trioxa-1,13-tridecanediamine and 1,11-diamino-3,6,9-trioxaundecane or a mixture of 4,7,10-trioxa-1,13-tridecanediamine, 4,9-dioxa-1,12-dodecanediamine and 1,11-diamino-3,6,9-trioxaundecane, and
• the at least bi-functional organic acid in the second formulation is maleic acid or itaconic acid or a mixture of maleic acid and itaconic acid, and
• the first formulation (a) has a pH of 7 to 12, and the second formulation (b) has a pH of 1.5 to 7, and
• the first formulation (a) is alkaline and has a pH greater than 7, and the second formulation (b) is acidic and has a pH of less than 7.

15. Kit for the strengthening of hair according to claim 14, wherein the kit further comprises a hair care conditioning formulation (c).

16. Kit for the strengthening of hair according to claim 14, wherein the concentration of the at least bifunctional Brönsted-Base in the first formulation is 1 - 30 % by weight.

17. Kit for the strengthening of hair according to claim 14, wherein the concentration of the at least bifunctional organic acid in the second formulation is 1 - 30 % by weight.

18. Kit for the strengthening of hair according to claim 14, wherein the first and the second formulations further contain at least a viscosity-modifying agent.

19. Kit for the strengthening of hair according to claim 14, wherein the first and the second formulations further contain at least one emulsifier and/or a surfactant.

20. Kit for the strengthening of hair according to claim 14, wherein the first and the second formulation further contain at least one preservative.

21. Kit for the strengthening of hair according to claim 14, wherein the first and the second formulation independently of each other comprise a cosmetically acceptable carrier and wherein the cosmetic acceptable carrier of the first formulation is either identical or different from the cosmetically acceptable carrier of the second formulation.

## Patentansprüche

1. Ein Verfahren zur Stärkung des Haares und zur Verringerung von Haarbruch, welches folgende Schritte umfasst:
a) Auftragen einer ersten Formulierung auf das Haar, wobei die erste Formulierung eine mindestens bifunktionelle Brønsted-Base der Formel X-R-Y aufweist, und
b) Auftragen einer zweiten Formulierung auf das Haar, wobei die zweite Formulierung eine mindestens bifunktionelle organische Säure enthält, die in der Lage ist, mit den Aminogruppen des Haares zu reagieren,
**dadurch gekennzeichnet, dass**
• die mindestens bifunktionelle Brønsted-Base der Formel X-R-Y in der ersten Formulierung 4,7,10-Trioxa-1,13-tridecandiamin oder ein Gemisch aus 4,7,10-Trioxa-1,13-tridecandiamin und 4,9-Dioxa-1,12-dodecandiamin oder ein Gemisch aus 4,7,10-Trioxa-1,13-tridecandiamin und 1,11-Diamino-3,6,9-trioxaundecan oder ein Gemisch aus 4,7,10-Trioxa-1,13-tridecandiamin, 4,9-Dioxa-1,12-dodecandiamin und 1,11-Diamino-3,6,9-trioxaundecan ist, und
• die mindestens bifunktionelle organische Säure in der zweiten Formulierung Maleinsäure oder Itaconsäure oder ein Gemisch aus Maleinsäure und Itaconsäure ist, und
• die erste Formulierung (a) einen pH-Wert von 7 bis 12 und die zweite Formulierung (b) einen pH-Wert von 1,5 bis 7 aufweist, und
• die erste Formulierung (a) alkalisch ist und einen pH-Wert von über 7 aufweist, während die zweite Formulierung (b) sauer ist und einen pH-Wert von unter 7 aufweist.

2. Das Verfahren gemäß Patentanspruch 1, wobei die Konzentration der mindestens bifunktionellen Brønsted-Base in Schritt (a) 1 - 30 Gew.-% beträgt.

3. Das Verfahren gemäß Patentanspruch 1, wobei die Konzentration der mindestens bifunktionellen organischen Säure in Schritt (b) 1 - 30 Gew.-% beträgt.

4. Das Verfahren gemäß den Patentansprüchen 1, 2, 3, wobei Schritt (a) vor Schritt (b) durchgeführt wird.

5. Das Verfahren gemäß den Patentansprüchen 1, 2, 3, wobei Schritt (b) vor Schritt (a) durchgeführt wird.

6. Das Verfahren gemäß den Patentansprüchen 1 bis 3, wobei das Haar zwischen den Schritten (a) und (b) getrocknet wird und die Trocknungsdauer 1 bis 60 Minuten beträgt.

7. Das Verfahren gemäß Patentanspruch 6, wobei eine Trockenvorrichtung zum Trocknen des Haars verwendet wird.

8. Das Verfahren gemäß den Patentansprüchen 1 bis 3, wobei die Formulierung aus Schritt (a) für 1 bis 45 Minuten auf dem Haar belassen wird.

9. Das Verfahren gemäß den Patentansprüchen 1 bis 3, wobei die Formulierung aus Schritt (b) 1 bis 45 Minuten lang auf dem Haar belassen wird.

10. Das Verfahren gemäß den Patentansprüchen 1 bis 3, wobei zwischen den Anwendungen der Schritte (a) und (b) oder (b) und (a) eine Wartezeit von 1 bis 60 Minuten eingehalten wird.

11. Das Verfahren gemäß den Patentansprüchen 1 bis 3, wobei die Formulierungen aus den Schritten (a) und (b) einzeln in einen für Kosmetik einsetzbaren Träger gemischt werden und wobei der für Kosmetik einsetzbare Träger der Formulierung aus Schritt (a) entweder identisch oder nicht identisch mit dem für Kosmetik einsetzbaren Träger der Formulierung aus Schritt (b) ist.

12. Das Verfahren gemäß den Patentansprüchen 1 bis 3, wobei die Formulierung aus Schritt (a) vor dem Auftragen auf das Haar in eine handelsübliche Haarfärbe- oder Haarbleichformulierung gemischt wird.

13. Das Verfahren gemäß den Patentansprüchen 1 bis 3, wobei das Haar vor der Anwendung von Schritt (a) mit einem thioglykolsäurehaltigen Haarpflegemittel behandelt wird.

14. Ein Kit zur Stärkung der Haare, das mindestens zwei getrennte Formulierungen (a) und (b) umfasst, wobei die erste Formulierung (a) eine mindestens bifunktionelle Brönsted-Base der allgemeinen Formel X-R-Y enthält und die zweite Formulierung (b) eine mindestens bifunktionelle organische Säure enthält, die in der Lage ist, mit den Aminogruppen des Haares zu reagieren, und wobei
• die mindestens bifunktionelle Brönsted-Base der Formel X-R-Y in der ersten Formulierung 4,7,10-Trioxa-1,13-tridecandiamin oder ein Gemisch aus 4,7,10-Trioxa-1,13-tridecandiamin und 4,9-Dioxa-1,12-dodecandiamin oder ein Gemisch aus 4,7,10-Trioxa-1,13-tridecandiamin und 1,11-Diamino-3,6,9-trioxaundecan oder ein Gemisch aus 4,7,10-Trioxa-1,13-tridecandiamin, 4,9-Dioxa-1,12-dodecandiamin und 1,11-Diamino-3,6,9-trioxaundecan ist, und
• die mindestens bifunktionelle organische Säure in der zweiten Formulierung Maleinsäure oder Itaconsäure oder ein Gemisch aus Maleinsäure und Itaconsäure ist, und
• die erste Formulierung (a) einen pH-Wert von 7 bis 12 und die zweite Formulierung (b) einen pH-Wert von 1,5 bis 7 aufweist, und
• die erste Formulierung (a) alkalisch ist und einen pH-Wert von über 7 aufweist, während die zweite Formulierung (b) sauer ist und einen pH-Wert von unter 7 aufweist.

15. Ein Kit zur Stärkung des Haares gemäß Patentanspruch 14, wobei das Kit des Weiteren eine Haarpflegeformulierung zur Konditionierung (c) enthält.

16. Ein Kit zur Stärkung des Haares gemäß Patentanspruch 14, wobei die Konzentration der mindestens bifunktionellen Brønsted-Base in der ersten Formulierung 1 - 30 Gew.-% beträgt.

17. Ein Kit zur Stärkung des Haares gemäß Patentanspruch 14, wobei die Konzentration der mindestens bifunktionellen organischen Säure in der zweiten Formulierung 1 - 30 Gew.-% beträgt.

18. Ein Kit zur Stärkung des Haares gemäß Patentanspruch 14, wobei die erste und die zweite Formulierung zusätzlich mindestens ein viskositätsveränderndes Mittel enthalten.

19. Ein Kit zur Stärkung des Haares gemäß Patentanspruch 14, wobei die erste und die zweite Formulierung außerdem mindestens einen Emulgator und/oder ein Tensid enthalten.

20. Ein Kit zur Stärkung des Haares gemäß Patentanspruch 14, wobei die erste und die zweite Formulierung außerdem mindestens ein Konservierungsmittel enthalten.

21. Ein Kit zur Stärkung des Haares gemäß Patentanspruch 14, wobei die erste und die zweite Formulierung unabhängig voneinander einen für Kosmetik einsetzbaren Träger umfassen und wobei der für Kosmetik einsetzbare Träger der ersten Formulierung entweder identisch oder nicht identisch mit dem für Kosmetik einsetzbaren Träger der zweiten Formulierung ist.

## Revendications

1. Une méthode pour renforcer les cheveux et réduire les cassures, comprenant les étapes de:
a) appliquer sur les cheveux une première formulation, la première formulation comprenant une base de Brønsted au moins bifonctionnelle de formule X-R-Y, et
b) appliquer sur les cheveux une seconde formulation, la seconde formulation comprenant un acide organique au moins bifonctionnel capable de réagir avec les groupes amines des cheveux,
**caractérisée en ce que**
• la base de Brønsted au moins bifonctionnelle de formule X-R-Y dans la première formulation est la 4,7,10-trioxa-1,13-tridécanediamine ou un mélange de 4,7,10-trioxa-1,13-tridécanediamine et de 4,9-dioxa-1,12-dodécanediamine ou un mélange de 4,7,10-trioxa-1,13-tridécanediamine et de 1,11-diamino-3,6,9-trioxaundécane ou un mélange de 4,7,10-trioxa-1,13-tridécanediamine, 4,9-dioxa-1,12-dodécanediamine et de 1,11-diamino-3,6,9-trioxaundécane, et
• l'acide organique au moins bifonctionnel dans la seconde formulation est l'acide maléique ou l'acide itaconique ou un mélange d'acide maléique et d'acide itaconique, et
• la première formulation (a) a un pH de 7 à 12, et la seconde formulation (b) a un pH de 1,5 à 7, et
• la première formulation (a) est alcaline et a un pH supérieur à 7, et la seconde formulation (b) est acide et a un pH inférieur à 7.

2. La méthode de la revendication 1, dans laquelle la concentration de la base de Brønsted au moins bifonctionnelle dans l'étape (a) est de 1 à 30 % en poids.

3. La méthode de la revendication 1, dans laquelle la concentration de l'acide organique au moins bifonctionnel dans l'étape (b) est de 1 à 30 % en poids.

4. La méthode des revendications 1, 2, 3 dans laquelle l'étape (a) est effectuée avant l'étape (b).

5. La méthode des revendications 1, 2, 3 dans laquelle l'étape (b) est effectuée avant l'étape (a).

6. La méthode des revendications 1 à 3, dans laquelle les cheveux sont séchés entre les étapes (a) et (b) et le temps de séchage est de 1 à 60 minutes.

7. La méthode de la revendication 6, dans laquelle un appareil de séchage est utilisé pour sécher les cheveux.

8. La méthode des revendications 1 à 3, dans laquelle la formulation de l'étape (a) est laissée sur les cheveux pendant 1 à 45 minutes.

9. La méthode des revendications 1 à 3, dans laquelle la formulation de l'étape (b) est laissée sur les cheveux pendant 1 à 45 minutes.

10. La méthode des revendications 1 à 3, dans laquelle il y a un temps d'attente entre les applications des étapes (a) et (b) ou (b) et (a) de 1 à 60 minutes.

11. La méthode des revendications 1 à 3, dans laquelle les formulations des étapes (a) et (b) sont mélangées séparément dans un support cosmétiquement acceptable et dans laquelle le support cosmétiquement acceptable de la formulation de l'étape (a) est soit identique soit différent du support cosmétiquement acceptable de la formulation de l'étape (b).

12. La méthode des revendications 1 à 3, dans laquelle la formulation de l'étape (a) est mélangée à une formulation de coloration ou de blanchiment des cheveux disponible dans le commerce avant l'application sur les cheveux.

13. La méthode des revendications 1 à 3, dans laquelle, avant l'application de l'étape (a), les cheveux sont traités avec une composition de soins capillaires contenant de l'acide thioglycolique.

14. Un kit pour le renforcement des cheveux comprenant au moins deux formulations séparées (a) et (b), dans lequel la première formulation (a) comprend une base de Bronsted au moins bifonctionnelle de la formule générale X-R-Y, et dans laquelle la seconde formulation (b) comprend un acide organique au moins bifonctionnel capable de réagir avec les groupes amine des cheveux, et dans laquelle
• la base de Brønsted au moins bifonctionnelle de formule X-R-Y dans la première formulation est la 4,7,10-trioxa-1,13-tridécanediamine ou un mélange de 4,7,10-trioxa-1,13-tridécanediamine et 4,9-dioxa-1,12-dodécanediamine ou un mélange de 4,7,10-trioxa-1,13-tridécanediamine et 1,11-diamino-3,6,9-trioxaundécane ou un mélange de 4,7,10-trioxa-1,13-tridécanediamine, 4,9-dioxa-1,12-dodécanediamine et 1,11-diamino-3,6,9-trioxaundécane, et
• l'acide organique au moins bifonctionnel dans la seconde formulation est l'acide maléique ou l'acide itaconique ou un mélange d'acide maléique et d'acide itaconique, et
• la première formulation (a) a un pH de 7 à 12, et la seconde formulation (b) a un pH de 1,5 à 7, et
• la première formulation (a) est alcaline et a un pH supérieur à 7, et la seconde formulation (b) est acide et a un pH inférieur à 7.

15. Kit pour le renforcement des cheveux selon la revendication 14, dans lequel le kit comprend en outre une formulation de conditionnement pour les soins capillaires (c).

16. Kit pour le renforcement des cheveux selon la revendication 14, dans lequel la concentration de la base de Bronsted au moins bifonctionnelle dans la première formulation est de 1 à 30 % en poids.

17. Kit pour le renforcement des cheveux selon la revendication 14, dans lequel la concentration de l'acide organique au moins bifonctionnel dans la seconde formulation est de 1 à 30 % en poids.

18. Kit pour le renforcement des cheveux selon la revendication 14, dans lequel la première et la seconde formulation contiennent en outre au moins un agent modificateur de viscosité.

19. Kit pour le renforcement des cheveux selon la revendication 14, dans lequel la première et la seconde formulation contiennent en outre au moins un émulsifiant et/ou un agent de surface.

20. Kit pour le renforcement des cheveux selon la revendication 14, dans lequel la première et la seconde formulation contiennent en outre au moins un agent de conservation.

21. Kit pour le renforcement des cheveux selon la revendication 14, dans lequel la première et la deuxième formulation comprennent indépendamment l'une de l'autre un support cosmétiquement acceptable et dans lequel le support cosmétiquement acceptable de la première formulation est soit identique soit différent du support cosmétiquement acceptable de la seconde formulation.
